# EUROPÄISCHE PATENTSCHRIFT

(11) **EP 0 824 896 B1**
(45) Veröffentlichungstag und Bekanntmachung des Hinweises auf die Patenterteilung: **05.11.2003**
(21) Anmeldenummer: 97113970.4
(22) Anmeldetag: 13.08.1997
(51) Int. Cl.: A61B 19/04, A41D 19/00

(54) **Gegenstand mit einer Trägerschicht aus Latex**
Article with a carrier layer made from latex
Article avec une couche porteuse en latex

(30) Priorität: 16.08.1996 AT 147796
(43) Veröffentlichungstag der Anmeldung: 25.02.1998
(73) Patentinhaber: Semperit Aktiengesellschaft, A-1031 Wien (AT)
(72) Erfinder: Schaller, Raimund, Dipl.-Ing. Dr., 2632 Wimpassing (AT); Wukovnig, Siegfried, Dr., A-2624 Breitenau am Steinfelde (AT)
(74) Vertreter: Secklehner, Günter, Dr.

(56) Entgegenhaltungen:
- EP-A- 0 681 912
- WO-A-91/03995
- DE-A- 1 915 589
- DE-A- 2 628 059
- US-A- 4 084 265
- US-A- 4 100 309
- US-A- 5 069 965

## Beschreibung

Die Erfindung beschreibt einen Gegenstand mit einer Trägerschicht aus Latex, der zumindest mit einem Teilbereich der Oberfläche zur Berührung oder zur Anlage mit einer menschlichen Haut ausgebildet ist, insbesondere-einen-Handschuh, ein Kondom öder dgl, der in diesem Teilbereich der Oberfläche mit einer Gleitschicht aus einem polymeren Material versehen ist sowie ein Verfahren zum Herstellen eines flexiblen Gegenstandes, insbesondere eines Handschuhes, eines Kondomes oder dgl., bei dem eine Trägerschicht aus Latex hergestellt wird und diese in zumindest einem Teilbereich ihrer Oberfläche mit einer Gleitschicht aus einem polymeren Material versehen wird und diese im Bereich ihrer von der Trägerschicht abgewendeten Oberfläche mit einer Rauheit versehen wird.

Viele an der Oberfläche unbehandelte, insbesondere flexible Gegenstände aus Kunststoff und Gummi, weisen einen relativ großen Reibungswiderstand gegenüber menschlicher Haut auf. So sind zum Beispiel unbehandelte Gummihandschuhe kaum anzuziehen. Um das Anziehen dieser Handschuhe zu erleichtern, wird konventionellerweise Handschuhpuder (z.B. Stärkepuder, Talkum, usw.) als Gleitmittel auf der Innenschicht des Handschuhes aufgebracht. Die Verwendung von Puder ist jedoch insbesonders im medizinischen Bereich problematisch, da Puderreste, falls sie in offene Wunden gelangen, zur Granulombildung führen können. Ein weiterer wichtiger Anwendungsbereich für puderfreie Produkte liegt in der Elektronikindustrie.

Eine klassische Methode, Gegenstände aus Gummi und im speziellen Handschuhe gegenüber der menschlichen Haut ohne Verwendung von Puder schlüpfrig zu machen, besteht in der Oberflächenchlorierung. Hierbei wird der Gegenstand mit wäßrigen, chlorgashaltigen Medien behandelt und anschließend gewaschen. Dabei wird vor allem eine gute Gleitfähigkeit gegenüber der trockenen menschlichen Haut erreicht, jedoch wird durch diese Oberflächenchlorierung die Polymerstruktur künstlich gealtert und es kommt so zu einer deutlichen Verschlechterung der physikalischen und chemischen Eigenschaften des Gegenstandes (Festigkeit, Reißdehnung, Wasserquellung, Lagerfähigkeit, etc.). Derartig oberflächenbehandelte Handschuhe sind jedoch mit nassen Händen nicht anziehbar.

Eine alternative Oberflächenbehandlung stellt die Beschichtung mit Hydrogelen dar. Einige solche Hydrogele sind schon seit langem bekannt. Dies sind z.B. Polyurethane, Polyvinylpyrrolidon, Polyhydroxyethylacrylat oder -methacrylat, Polyhydroxypropylacrylat oder -methacrylate und Copolymere miteinander oder mit Acryl- oder Methacrylsäure, Acryl- oder Methacrylester oder Vinylpyridin.

Eine derartige Beschichtung ist aus der US 3,813,695 A bekannt, die einen getauchten Gummihandschuh beschreibt, der an der Innenseite mit einem-Hydrogelpolymer, wie z.B. Polyvinylpyrrolidon, Polyhydroxyethylacrylat oder -methacrylat, Polyhydroxypropylacrylat odermethacrylat und Copolymere dieser untereinander oder mit Acryl- oder Methacrylsäure. Das bevorzugte Hydrogelpolymer ist ein Copolymer von 2-Hydroxyethylmethacrylat mit Methacrylsäure oder mit 2-Ethylhexylacrylat oder einem ternärem Copolymer von 2-Hydroxyethylmethacrylat, Methacrylsäure und 2-Ethylhexylacrylat.

Ein bekanntes Herstellungsverfahren ist aus der US 4,482,577 A bekannt. Bei diesem wird die Beschichtung eines flexiblen, vulkanisierten Operationshandschuhs mit einem hydrophilen Polymer geoffenbart, wodurch die Aufbringung von Puder an der Handschuhinnenseite unterlassen werden kann. Das hierbei verwendete Copolymer besteht aus einer Mischung von 2-Hydroxyethylmethacrylat und 2-Ethylhexylacrylat.

Ein anderes Verfahren gemäß US 4,100,309 A legt die Aufbringung einer gleitfähigen Schicht aus einem Polyurethan-Polyvinylpyrrolidon-Komplex offen.

Üblicherweise wird bei diesen Produkten bzw. Verfahren das Hydropolymer gemeinsam mit einem Härtungsmittel in gelöster Form auf das Produkt aufgebracht, anschließend das Lösungsmittel durch Trocknung entfernt, wobei die Polymerschicht ausgehärtet und so eine Hydrogelschicht ausgebildet wird.

Weitere bekannte Lösungen offenbaren den Einsatz von Vinylidenhalogenid- bzw. Vinylhalogenidlatices zur Herstellung einer Gleitschicht auf Gummiartikel, wie z.B. US 5,069,965 A, wo die Gleitfähigkeit meist chlorhaltiger Polymere ausgenutzt wird.

Der Einsatz von diversen Latexgemischen für eine Gleitschichtherstellung ist aus der DE 26 28 059 C und der US 4,082,826 A bekannt. Hier kommen insbesondere Gemische aus mindestens zwei Latextypen zum Einsatz, wobei ein Latextyp mit einer hohen Dehnung als Haftvermittler und ein Latextyp mit hoher Härte bzw. geringer Dehnung zum Erhalt einer ausreichenden Gleitfähigkeit dient. Diese Rezepturen sind üblicherweise mehr oder minder gute Kompromisse zwischen Gleitfähigkeit, ausreichender Filmflexibilität und Haftung des Films am Gummiprodukt. Insbesondere beim Einsatz der Gleitschicht bei sehr flexiblen und hoch dehnbaren Gummiartikeln gibt es hier oft Probleme, daß die eingesetzten Gleitschichten entweder beim Dehnen sich vom Untergrund zu lösen beginnen bzw. die Gleitfähigkeit in der Praxis nicht befriedigend ist.

In dem Veifahren nach der EP 0681 912 A2 wird die Gleitschicht aus einem Copolymerlatex gebildet, welcher mit Hilfe eines Koagulationsschrittes auf die Trägerschicht fixiert wird. Der Latex besteht aus einem Copolymeren, das einen wesentlichen Anteil an hydrophilen Monomerbausteinen enthält.

Einen anderen Weg zur Erzeugung von Hautschlüpfrigkeit bei puderfreien medizinischen Naturlatexhandschuhen wird in der US 4,143,109 A beschrieben. Dort werden in einer Trägerschicht gebundene Maisstärke- oder Polyethylenteilchen eingelagert, wobei die Trägerschicht dünner als der Teilchendurchmesser ist und die Teilchen aus der Oberfläche herausragen. Die so gebundenen Puderpartikel bewirken eine ausreichende Gleitfähigkeit der Handschuhinnenseite. Probleme ergeben sich bei dieser Lösung zur Erreichung von Hautschlüpfrigkeit bei größerer Dehnung der Produkte, was beim Anziehen von medizinischen Latexhandschuhen unvermeidlich ist: Die oberflächlich gebundenen Partikel lösen sich leicht ab und die Puderfreiheit ist nicht mehr gewährleistet.

Alle oben beschriebenen puderfreien Artikel sind nur unter genauestem Einhalten der zugehörigen Prozeßbedingungen und Materialrezepturen mit befriedigenden Produkteigenschaften herstellbar. Während eine gute Gleitfähigkeit der Oberflächen gegenüber trockener Haut meist leicht erreicht wird, gibt es sehr oft Schwierigkeiten in der Reproduzierbarkeit hinsichtlich Naßgleitfähigkeit. Insbesondere bei dünnwandigen flexiblen Artikeln, welche auch unter hohen Dehnungen gute Gleitfähigkeiten aufweisen müssen, bedarf es oft jahrelanger Feinoptimierung seitens der Hersteller, welche meist Kompromisse seitens diverser Produkteigenschaften eingehen müssen. So wird vielfach trotz Einsatz von polymeren Gleitschichthydrogelen zusätzlich die Oberfläche leicht chloriert. Andere Hersteller verwenden trotz patentierter Polymergleitschichten zusätzlich geringe Mengen von Handschuhpuder. Wieder andere Produzenten sehen sich mit laufenden Schwankungen hinsichtlich der Naßgleitfähigkeit ihrer Produkte konfrontiert.

Die EP 0 681 912 A beschreibt flexible Gummiartikel, auf welchen eine Gleitschicht angebracht ist, die die Schlüpfrigkeit gegenüber der menschlichen Haut gewährleistet. Diese besteht aus einem koagulierten Copolymerlatex, welcher aus einem Copolymeren besteht, das einen hydrophilen Anteil als Monomerbaustein enthält. Der hydrophile Anteil besteht vorzugsweise aus Acrylsäure bzw. Methacrylsäure oder aus Acrylaten bzw. Methacrylaten, bzw. einem Gemisch diese Monomerbausteine bzw. können auch andere hydrophile bzw. polare Polymerbausteine verwendet werden. Daneben können noch oberflächenaktive Stoffe und/oder Siloxane in der Gleitschicht verwendet werden, wodurch einerseits eine verbesserte Naßanziehbarkeit, z.B. bei Handschuhen, erreicht wird und andererseits bei diversen Gummiartikeln, wie z.B. Handschuhe oder Kondome, durch die Verwendung von Tensiden mit bakteriziden und/oder fungiziden Eigenschaften ein verbesserter Infektionsschutz für den Anwender erreicht werden kann. Als Latex kann sowohl Naturlatex als auch Syntheselatex verwendet werden.

Aus der DE 26 28 059 A sind Gegenstände aus Gummi, z.B. Gummihandschuhe, mit einem dehnbaren, gleitfähigen polymeren Kunstharzüberzug bekannt. Damit sollen die mit puderförmigen Trennmitteln verbundenen Nachteile vermieden werden, wobei das aus Kunstharz gebildete Trennmittel gleichzeitig als dehnbares, gleitfähiges Überzugsmaterial zur Erniedrigung des Reibungskoeffizienten herangezogen werden kann. Das Kunstharz kann unter anderem durch Mischpolymerisate aus Acrylaten und (Poly) Vinylchlorid, Acrylkautschukpolymeren, etc. gebildet sein. Bevorzugt wird dieser Kunstharzüberzug sowohl auf der äußeren als auch auf der inneren Oberfläche der Gummigegenstände aufgebracht.

Die DE 19 15 589 A beschreibt einen Handschuh sowie ein Verfahren und eine Vorrichtung zur Herstellung des Handschuhes aus Kautschuk mit hoher Gleitfähigkeit der Innenfläche. Wiederum wird aufgrund der höheren Gleitfähigkeit die Anziehbarkeit der Handschuhe verbessert, indem die Kautschukoberfläche mit freiem Chlor aufgerauht wird und dabei ein Rauhigkeitsgrad von mindestens 5µm, vorzugsweise von 40-150 µm, durch die Rauhigkeit der Außenfläche der Tauchform erzeugt wird. Als Material für den Handschuh wird Kautschuk verwendet.

Aus der US 4,084,265 A sind Schutzhandschuhe bekannt, welche aus zwei Kunstofffilmen, wie beispielsweise Polyethylen, gebildet werden. Die beiden Filme werden dazu miteinander verschweißt und wird den Handschuhen, d.h. zumindest einem der Filme, zur Verringerung der Adhäsion eine entsprechende Oberflächenstrukturierung gegeben. Die Ausbildung der Oberflächenstruktur erfolgt dabei mit Hilfe eines Walzensystems, insbesondere Reliefrollen und wird in zumindest einen der Filme geprägt.

Die US 4,100,309 A beschreibt Gegenstände, wie z.B. Katheter, Kondome oder Peristaltikpumpen, welche aus einem Substrat bestehen, das mit einem Polyvinylpyrollidon-Polyurethan- Interpolymer beschichtet ist. Zur Herstellung wird ein Polyisocyanat und ein Polyurethan in einem Lösungsmittel, wie z.B. Metyl-Ethyl-Keton, auf das Substrat aufgetragen und in der Folge das Lösungsmittel abgedampft. Als Substrat selbst kann ein Polyurethan verwendet werden, wobei in diesem Fall lediglich Polyisocyanat in weiterer Folge notwendig ist. Es wird damit ebenfalls durch die Verringerung des Reibungskoeffizienten insbesondere auch die Naßanziehbarkeit verbessert.

Der vorliegenden Erfindung liegt die Aufgabe zugrunde, Gegenstände aus Kunststoff und/oder Gummi herzustellen, die eine gute Schlüpfrigkeit gegenüber der menschlichen, insbesondere nassen Haut und eine ungestörte Reproduzierbarkeit aufweisen.

Diese Aufgabe der Erfindung wird durch einen Gegenstand gelöst, bei dem zumindest eine Teilfläche des Teilbereiches eine, bevorzugt regelmäßig wiederkehrende, Gestaltsabweichung der Oberfläche aufweist, wobei das Verhältnis der Abstände der Gestaltsabweichung zu ihrer Tiefe zwischen 100 : 1 und 5 : 1 liegt und die gemittelte Rauhtiefe zwischen 0,5 µm und 100 µm, bevorzugt zwischen 1 µm und 40 µm beträgt. Von Vorteil ist hierbei, daß die flexiblen Produkte mit leicht gerauhten Polymeroberflächen eine wesentlich bessere Schlüpfrigkeit gegenüber der menschlichen Haut aufweisen als glatte Gleitschichten. Dies gilt insbesondere für die oft schwer erreichbare Naßgleitfähigkeit. Ein weiterer Vorteil der vorliegenden Erfindung ist, daß durch die Vermeidung von okklusivem Hautkontäkt der Tragekomfort, der Abtransport und gegebenenfalls die Sorption von Schweiß wesentlich verbessert wird. Wesentlich ist auch, daß durch die rigorose Verringerung der Kontaktfläche zwischen Haut und Polymer in vielen Fällen eine wesentlich verbesserte Hautverträglichkeit zu erwarten ist. Zusätzlich werden allergische Reaktionen verringert und es entsteht kein Engegefühl nach Überziehen der Produkte. Mit erfindungsgemäß gerauhter Oberflächenmorphologie kann eine gute Gleitfähigkeit auch mit weniger optimalen Gleitschichtpolymeren erreicht werden. Es zeigt sich, daß meist die optimale Oberflächenrauhheit wichtiger ist als die chemische Zusammensetzung der Gleitschicht. De facto kann mit einer Unzahl an Polymeren und Polymerkombinationen, welche mit glatter Oberfläche nur eine schlechte Gleitfähigkeit gegenüber der menschlichen Haut aufweisen, bei Einhaltung einer gerauhten Oberflächenstruktur eine gute Schlüpfrigkeit sowohl im nassen als auch im trockenen Zustand erreicht werden.

Vorteilhaft ist eine Weiterbildung nach Anspruch 2, da dadurch extrem dünne und extrem dehnbare sowie stark belastbare Gegenstände herstellbar sind.

Die Qualität der Gegenstände kann durch die Anwendung der Merkmale nach den Ansprüchen 3 oder 4 erheblich verbessert werden.

Durch die weitere Ausgestaltung nach Anspruch 5 kann der Schweißhaushalt an der Hautoberfläche im Bereich der gerauhten Oberflächen in den Teilbereichen besser gesteuert werden, da der Handschuh eine höhere Menge an Feuchtigkeit aufnehmen kann.

Eine gute Ausgewogenheit zwischen Strapazierfähigkeit und einer guten Hautverträglichkeit wird durch die Ausgestaltung nach Anspruch 6 oder 7 erzielt.

Ein hoher Tragekomfort bzw. eine hohe Benutzerfreundlichkeit wird bei Gegenständen durch die Verwendung eines Polymers durch Anspruch 8 erzielt.

Eine gute Verbindung mit der Gleitschicht in den Teilbereichen wird durch die Weiterbildung nach Anspruch 9 erzielt.

Vorteilhaft ist es für die Widerstandsfähigkeit der Trägerschicht, diese nach Anspruch 10 auszubilden.

Von Vorteil ist die Ausbildung des Gegenstandes gemäß Anspruch 11, da damit die Rauheit der einzelnen Oberflächen bzw. Teilbereiche an die unterschiedlichen Einsatzbereiche des Gegenstandes einfach anpaßbar ist.

Ein angenehmes Gefühl auf der Haut hinterläßt ein Gegenstand, wenn die Gleitschicht gemäß Anspruch 12 oder 13 ausgebildet ist.

Gute Eigenschaften für die Gleitschicht und ein festes Anhaften derselben an der Trägerschicht wird durch die weitere Ausführungsvariante gemäß Anspruch 14 erreicht.

Eine gute Hautverträglichkeit für Gegenstände wird bei einer Ausführungsform nach den Ansprüchen 15 bzw. 16 erreicht.

Besonders angenehme Eigenschaften bei eine starke Schweißbildung aufweisenden Personen wird für Gegenstände bei der Weiterbildung nach Anspruch 17 erzielt.

Widerstandsfähige Gleitschichten können durch die Ausgestaltung nach Anspruch 18 erreicht werden.

Es ist auch eine Ausbildung nach Anspruch 19 möglich, wodurch die Naßschlüpfrigkeit der Gegenstände zusätzlich verbessert werden kann.

Bevorzugt ist eine Ausgestaltung nach Anspruch 20, da dadurch eine so dünne, die Gleitfähigkeit zusätzlich erhöhende Schicht geschaffen wird, bei der gleichzeitig ein Verkleben der Vertiefüngen verhindert ist.

Von Vorteil ist bei der Ausgestaltung nach Anspruch 21, daß damit die Schlüpfrigkeit und die Gleiteigenschaften sowie eine entsprechende Rauheit, beispielsweise das Erfassen von Arbeitswerkzeugen bzw. Gegenständen bei der Montage erzielt werden können.

Vorteilhaft ist eine Ausführungsvariante nach Anspruch 22 oder 23, da dadurch die Wirkung der Rauheit im Bereich der Gleitschicht noch verstärkt bzw. in jenen Bereichen, in denen keine Aufrauhung erfolgt, zumindest eine bedingte Verbesserung der Gleiteigenschaften erzielt wird.

Eine bevorzugte Ausgestaltung des Gegenstandes ist in Anspruch 24 gekennzeichnet.

Die Erfindung umfaßt auch das Verfahren zur Herstellung eines flexiblen Gegenstandes, insbesondere eines Handschuhes, bei dem die Oberfläche zumindest in Teilflächen des Teilbereiches mit bevorzugt regelmäßig wiederkehrenden Gestaltsabweichungen versehen wird, bei welchen das Verhältnis der Abstände der Gestaltsabweichungen zu ihrer Tiefe zwischen 100: 1 und 5 : 1 liegt und deren gemittelte Rauhtiefe zwischen 1 µm bis 100 µm, bevorzugt 1 bis 40 µm, beträgt.

Durch diese Maßnahmen wird die erfindungsgemäße Aufgabe gelöst und wird dadurch die Gleitfähigkeit solcher Gegenstände auf der menschlichen Haut, insbesondere die Naßgleitfähigkeit, erheblich verbessert.

Die Herstellung der Rauheit in der Trägerschicht und/oder der Gleitschicht bzw. deren Teilflächen kann durch das Vorgehen nach den Ansprüchen 26 bis 29 verbessert werden.

Weitere Verbesserungen der Gegenstände werden durch die Maßnahmen nach den Ansprüchen 30 bis 33 erzielt.

Schließlich ist es auch vorteilhaft, nach Anspruch 34 vorzugehen, da dadurch auch die Handhabung von Werkzeugen bzw. Maschinenteilen oder Bauteilen, insbesondere dann, wenn diese Handschuhe bei der Montage von elektronischen Komponenten oder Reinraumteilen verwendet werden, verbessert wird.

Die Erfindung wird im nachfolgenden anhand der in den Zeichnungen dargestellten Ausführungsbeispiele näher erläutert.

Es zeigen:
- Fig. 1: einen erfindungsgemäßen Gegenstand mit einer Gleitfläche und einer entsprechenden Rauheit in Ansicht, geschnitten, in vereinfachter, schematischer Darstellung;
- Fig. 2: eine andere Ausführungsvariante eines erfindungsgemäßen Gegenstandes in der Art eines Handschuhes in Draufsicht, geschnitten;
- Fig. 3: den Handschuh nach Fig. 2 in Stirnansicht, geschnitten und vereinfachter, schematischer Darstellung;
- Fig. 4: einen erfindungsgemäßen Verfahrensablauf zur Herstellung eines Gegenstandes in vereinfachter, schematischer Darstellung;
- Fig. 5: eine Behandlungsvorrichtung zum Herstellen der Rauheit auf einer Oberfläche des Handschuhs in Stirnansicht, geschnitten, gemäß den Linien V - V in Fig. 4:
- Fig. 6: eine andere Ausführungsvariante einer Behandlungsvorrichtung für den erfindungsgemäßen Verfahrensablauf nach Fig. 4 in Stirnansicht, geschnitten;
- Fig. 7: eine Stirnansicht einer Porzellanform, geschnitten mit den darauf aufgebrachten Lagen zur Herstellung eines Handschuhes in vereinfachter, schematischer Darstellung.

Einführend sei festgehalten, daß in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Weiters können auch Einzelmerkmale aus den gezeigten unterschiedlichen Ausführungsbeispielen für sich eigenständige, erfindungsgemäße Lösungen darstellen.

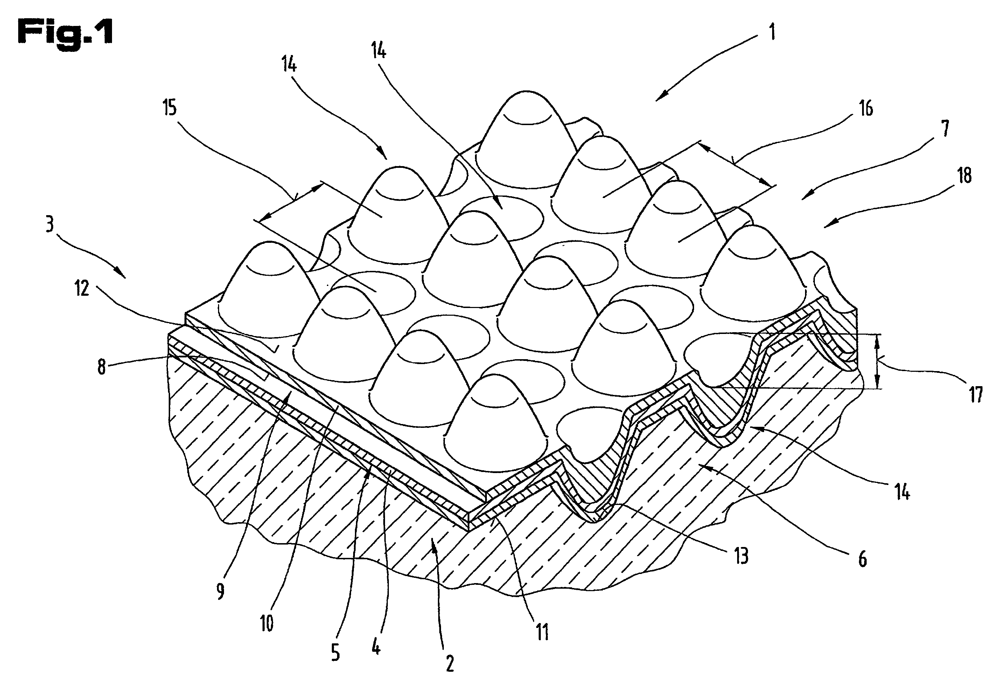

In der Fig. 1 ist ein erfindungsgemäßer, bevorzugt flexibler Gegenstand 1 aus einer Latexmischung 2 gezeigt. Dieser besteht aus einem Trägermaterial, das, wie dies bei der Herstellung von dünnwandigen Gummi- und Kunststoffartikeln üblicherweise der Fall ist, bevorzugt durch ein mehrmaliges Tauchverfahren gebildet wird. Bei diesen Gegenständen 1 kann es sich um Handschuhe 3, beispielsweise medizinische Operations- und Untersuchungshandschuhe, Katheder, Kondome, Fingerlinge, Badehauben, Schwimmflossen oder Schutzhandschuhe für die Arbeit in Reinraumbereichen handeln. Für diese Herstellung von dünnwandigen, insbesondere elastischen Gummi- und/oder Kunststoffartikeln sind nachstehende Tauchverfahren bekannt:

Tauchmischungen zur Herstellung von dünnwandigen Kunststoffartikeln bestehen beispielsweise aus im Weichmacher (z.B. Dioctylphtalat, Diisononylphtalat, u.a.) emulgiertem feinstem PVC-Pulver. Hier wird die Polymerschicht durch Tauchen auf eine Positivform aufgetaucht und in nachfolgenden Heißluftöfen geliert, wobei der Weichmacher in die PVC-Partikel dringt und eine homogene, flexible Schicht aus Weich-PVC erzeugt. Die Tauchartikel werden üblicherweise vor der völligen Ausgelierung mit Baumwollflocken oder nach vollständiger Ausgelierung mit puderförmigem Trennmittel behandelt, um einerseits die Artikel gut entformen zu können und andererseits eine befriedigende Hautschlüpfrigkeit zu erreichen. Des weiteren wird durch die Verwendung von Flocken oder Puder die Absorption bzw. der Abtransport von Schweiß wesentlich gefördert und dadurch der Tragekomfort und die Hautverträglichkeit wesentlich erhöht.

Dünnwandige Gummiartikel, wie z.B. der Handschuh 3 werden vorzugsweise durch Tauchen einer Form in der Latexmischung 2 erhalten, wobei die Latexmischung 2 sowohl auf Naturlatex als auch auf Syntheselatex basieren kann. Die verwendeten Latexmischungen 2 können sowohl unvernetzt als auch im vorvernetzten Zustand vorliegen. Im Falle einer unvernetzten oder nur partiell vorvernetzten Latexmischung 2 muß die erhaltene Gummischicht noch an der Tauchform vulkanisiert werden, wodurch eine Trägerschicht 4 gebildet wird.

Vorzugsweise werden Latexmischungen 2 verwendet, die vernetzte oder größtenteils vorvernetzte Polymere 5 enthalten. Diese Latexmischung 2 wird auf eine Tauchform 6, welche die Gestalt des Endproduktes vorgibt, aufgebracht und koaguliert und erzeugt so eine dünne Schicht, nämlich die Trägerschicht 4, aus elastischem, relativ resistentem Gummimaterial. Die Latexmischung 2 enthält die üblichen Compoundierungszutaten, wie beispielsweise Schwefel, Zinkoxid, organische Beschleuniger (u.a. Zinksalze von Dithiocarbamaten, Thiurame, Thioharnstoff, etc.), Stabilisatoren, Wachse, Alterungsschutzmittel, Viskositätsregler, Füllstoffe, Farben, usw.. Als Latexmischung 2 für die Trägerschicht 4 kann sowohl Naturkautschuk, als auch Synthesekautschuk, der für die Anwendung in Tauchverfahren geeignet ist, verwendet werden. Von den natürlichen und synthetischen Latices werden vorzugsweise Naturkautschuk, Polychloropren, synthetisches Polyisopren, Nitrilbutadien- und Styrolbutadienkautschuk bzw. eine Mischung dieser Polymere verwendet.

Nach erfolgter Tauchung wird die mit der Latexschicht belegte Tauchform 6 üblicherweise durch Trockenstrecken geführt, gewaschen und der Latexfilm in Heißluftöfen vulkanisiert.

Auf diese Trägerschicht 4 wird dann zumindest in einem Teilbereich 7 einer Oberseite 8 der Trägerschicht 4 eine Gleitschicht 9 aus polymeren Material 10 im Zuge des vorhin umrissenen Tauchprozesses durch Tauchen oder Sprühen in einem oder mehreren Schritten auf den Gegenstand 1 fixiert. Im speziellen Fall der Herstellung von Tauchartikel, wie beim Gegenstand 1, wird deren Oberfläche üblicherweise in die Tauchformseite und die Tauchbadseite unterteilt. Die Tauchformseite ist im vorliegenden Ausführungsbeispiel als Unterseite 11 bezeichnet und liegt an der Tauchform 6 an, die beispielsweise aus Porzellan, Kunststoff oder ähnlichem bestehen kann.

Die Tauchbadseite wird entweder durch die Oberseite 8 der Trägerschicht 4 oder die Oberfläche 12 der Gleitschicht 9 gebildet. Im vorliegenden Fall ist eine Oberfläche 13 der Tauchform 6 mit bevorzugt regelmäßig wiederkehrenden Gestaltsabweichungen 14 versehen. Diese werden durch Gestaltsabweichungen 14, beispielsweise Vertiefungen in der Oberfläche 13 oder durch über die Oberfläche 13 vorragende Erhebungen, gebildet. Ein Verhältnis von Abständen 15, 16 zu einer Tiefe 17 derselben liegt zwischen 100 : 1 und 5 : 1. Damit entspricht die Rauheit der Unterseite 11 des Gegenstandes 1 der Morphologie der Tauchform 6.

Wird, wie nachfolgend noch im Detail erläutert wird, sichergestellt, daß eine Schichtdicke der Trägerschicht 4 sowie der auf dieser aufgebrachten Gleitschicht 9 in den Teilbereichen 7 über einen Großteil des Gegenstandes 1 gleichgehalten, so bildet sich auch eine entsprechende Rauheit auf der der Tauchbadseite zugewandten Oberfläche 12 der Gleitschicht 9 aus. Diese Tauchbadseite bildet üblicherweise die Innenseite des Produkts, wenn es sich beispielsweise um hüllenförmige Gegenstände 1 bzw. dünnwandige Gummiprodukte handelt, da beim Abziehen der Gegenstände 1 von der Tauchform 6 die Produkte gewendet werden. Üblicherweise ist die Oberfläche 12 jene Seite des Gegenstandes 1, die mit der Haut des Benutzers in Berührung kommt und dementsprechend ausgebildet sein soll, um Irritationen der Haut bzw. die Schweißabsonderung ohne Störung der Haut zu ermöglichen. Beim vorliegenden Gegenstand 1 ist vorgesehen, daß entweder die Tauchformseite oder Tauchbadseite oder beide mit einer entsprechenden Rauheit ausgestattet werden können. Dementsprechend ist es auch möglich, daß auf einer oder beiden Seiten in Teilbereichen 7, aber selbstverständlich auch über die gesamte Oberseite 8 bzw. Unterseite 11, eine Gleitschicht 9 angeordnet sein kann.

Im Rahmen der Erfindung ist es natürlich auch möglich, Gegenstände 1, vor allem wenn es sich um dünnwandige Artikel handelt, die hautschlüpfrig sein sollen, durch ein Spritzgußverfahren herzustellen. Derartige Verfahren werden vor allem für die ABC-Schutzausrüstungen aus Butylkautschuk, hier vor allem für die Handschuhe 3 verwendet.

Durch die zumindest in Teilbereichen 7 auf der Oberseite 8 der Trägerschicht 4 angeordnete Gestaltsabweichungen 14 in der Gleitschicht 9 kann der Schweißhaushalt geregelt und der Tragekomfort erhöht werden.

Die Kontaktfläche zwischen Haut und Gleitschicht 9 bzw. dem Polymer soll möglichst gering gehalten werden, um ein Festkleben an der menschlichen Haut zu verhindern, wodurch unter anderem die Gefahr von Hautirritationen und unter Umständen allergischen Reaktionen minimiert werden. Des weiteren wird durch die erfindungsgemäß ausgebildete Oberfläche 12 eine gute Gleitfähigkeit der flexiblen Gegenstände gegenüber der menschlichen Haut und daraus resultierend eine gute Anziehbarkeit im nassen und trockenen Zustand erreicht.

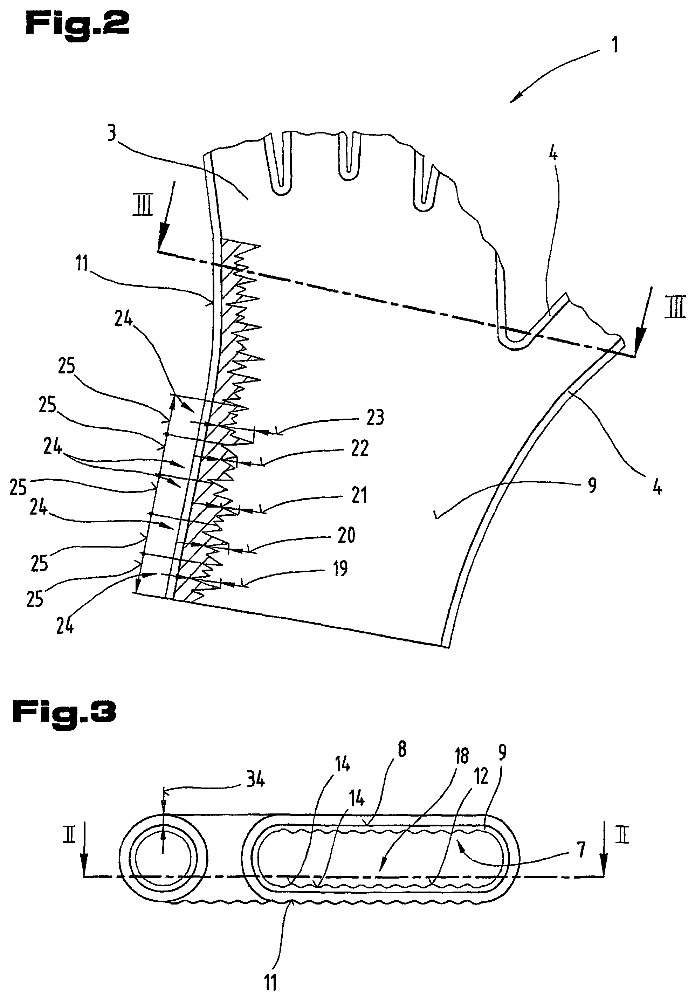

Wie besser anhand der Fig. 2 und 3 und hier wiederum bei einem durch einen Handschuh 3 gebildeten Gegenstand 1 gezeigt ist, besteht dieser wiederum aus der Trägerschicht 4 und einer Gleitschicht 9, die auf die gesamte innere Oberfläche 8 der Trägerschicht 4 aufgebracht ist. Wie dieser Darstellung weiters zu entnehmen ist, sind zwei Teilbereiche 7, 18 und zwar jeweils die etwa einer Handinnenseite bzw. einem Handrücken zugewandten Teilbereiche mit den bevorzugt regelmäßig wiederkehrenden Gestaltsabweichungen 14 versehen, deren Verhältnis zwischen den Abständen 15, 16 der Gestaltsabweichung 14 und der Tiefe, wie bereits dem Ausführungsbeispiel in Fig. 1 erläutert, zwischen 100 : 1 und 5 : 1 liegt. Dazu beträgt eine gemittelte Rauhtiefe zwischen 0,5 µm und 100 µm, bevorzugt zwischen 1 µm und 40 µm. Diese gemittelte Rauhtiefe stellt das arithmetische Mittel aus Einzelrauhtiefen 19 bis 23 dar, die in fünf aneinandergrenzenden Einzelmeßstrecken 24 gleicher Länge 25 ermittelt werden. Dadurch wird erreicht, daß ein ziemlich einheitliches Haftungsvermögen bzw. Gleitvermögen zwischen den einzelnen Bereichen der Oberfläche 12 bzw. falls auch die Unterseite 11 mit einer Gleitschicht 9 beschichtet ist, auf der dieser vorgeschalteten Oberfläche erreicht wird, wodurch die Schlüpfrigkeit des Artikels und vor allem auch die Naßschlüpfrigkeit erheblich verbessert werden kann.

Die erfindungsgemäße Schicht kann aus einer Vielzahl an gängigen flexiblen Polymerwerkstoffen hergestellt werden, wobei die verwendeten Polymere vor ihrer Verarbeitung sowohl in Latexform als auch in gelöster oder reiner Form vorliegen können. Bei der Auswahl von geeigneten Trägermaterialien für erfindungsgemäße Artikel (z.B. vorvernetzter NBR-Latexprodukte) kann die erforderliche Gleitwirkung auch alleine durch die gerauhte Oberflächenmorphologie erreicht werden.

Vorzugsweise besteht die erfindungsgemäße Oberfläche 12 bzw. die Gleitschicht 9 aus einem leicht vernetzten Polymeren, welches stark hydrophile Gruppen enthält. Diese bestehen beispielsweise aus Acrylsäure bzw. Methacrylsäure, aus Acrylaten bzw. Methacrylaten, bzw. aus einem Gemisch dieser Monomerbausteine des Polymers, wobei auch andere stark hydrophile bzw. polare Polymerbausteine, wie z.B. Vinylpyrollidon, Vinylalkohol gut geeignet sind. Zusätzlich können auch hydrophobe bzw. weniger wasseraufnehmende Anteile im Polymer vorhanden sein, vorzugsweise Styrol, Butadien, Isopren und/oder Acrylnitril. Des weiteren können auch mit einer Vielzahl von Polyurethanen die erwünschten Eigenschaften erreicht werden. Unterschiedlich ist hierbei zu den bisher bekannten Verfahren, daß die erfindungsgemäßen Produkteigenschaften vorwiegend über die Rauhheit der Oberfläche 12 bzw. Unterseite 11 erreicht werden, wobei mit steigender gemittelter Rauhtiefe bis etwa 40 µ Rauhtiefe die Schlüpfrigkeit gegenüber der menschlichen Haut steigt und die Klebrigkeit der Polymeroberflächen abnimmt.

Wird die Oberfläche 12 bzw. Unterseite 11, welche die äußere Oberfläche des Handschuhs 3 bildet, weiter aufgerauht, tritt bei den meisten Produkten abhängig von Härte und Dehnungsmodul schrittweise ein subjektiv unangenehm reibendes Gefühl auf. Bei Rauhtiefen über 100 µm kommt es meist zu einer wesentlichen Erhöhung der Polymer-Polymerreibung und eventuell bei reibendem Kontakt mit menschlicher Haut zu mechanisch bedingten Irritationen. Die hier in ihrer Abhängigkeit von der gemittelten Rauhtiefe beschriebenen Oberflächeneigenschaften sind natürlich auch von Art und Herstellungstyp der Rauheit sowie von Polymer- und Produkttyp abhängig und müssen für jeden Polymer- und Produkttyp einzeln optimiert werden. Vorteilhaft ist dabei, daß durch die gemittelte Rauhtiefe, die über die Teilbereiche 7 bzw. 18 gleich ist, einzelne Irritationszonen in den Teilbereichen 7, 18, die den positiven Gesamteffekt der erfindungsgemäßen Lösung nachteilig beeinflussen können, grundsätzlich vermieden werden.

Selbstverständlich kann es bei sehr weichen und klebrigen bzw. unvernetzten Polymertypen (z.B. diverse Acrylate bzw. Polyurethane, schwach oder unvernetztem Naturkautschuk) auch bei Aufbringung einer Oberflächenrauheit zu Verklebungen bzw. nur geringer Gleitfähigkeit gegenüber menschlicher Haut kommen. Bei Vorhandensein einer rauhen Unterseite 11 undtoder Oberfläche 12 des Gegenstandes 1 sind diese Verklebungen jedoch wesentlich geringfügiger. Umgekehrt können in Gegenwart erfindungsgemäßer Oberflächenrauheiten auch mit relativ weichen Polymeren (z.B. Polyacrylaten) gute Gleitfähigkeiten gegenüber der menschlichen Haut erreicht werden, wobei bei konventionell glatten Gleitschichten mit denselben Polymeren keine Hautschlüpfrigkeit vorhanden ist. Durch den Umstand, daß bei erfindungsgemäßen Oberflächenschichten weichere Polymere mit höheren Zugdehnungen eingesetzt werden können, verringern sich bei dünnwandigen Gegenständen 1 mit hoher Dehnfähigkeit die Haftungsprobleme der Oberflächenschicht beim Dehnen derselben drastisch. Des weiteren wird die Auswahl des Gleitschichtpolymers wesentlich vereinfacht, da der Haupteinflußfaktor für Tragekomfort und Schlüpfrigkeit erfindungsgemäß durch die Oberflächenmorphologie bestimmt wird.

Die erwähnten erfindungsgemäßen Gleitschichtpolymere werden vorzugsweise durch Sprühen, Tauchen, Streichen oder andere geeignete Verfahren auf die Oberfläche des Gummiartikels oder der Tauchform gebracht.

Der in den Fig. 2 und 3 dargestellte Gegenstand 1 kann beispielsweise als puderfreier Latexuntersuchungshandschuh ausgebildet sein. Zur Herstellung von Latexhandschuhen werden üblicherweise leicht rauhe Tauchformen 6 aus Porzellan mit einer gemittelte Rauhtiefe zwischen 0,5 µm und 100 µm, bevorzugt 1µm bis 40 µm, verwendet, die in eine wäßrige Koagulationslösung, welche Kalziumnitrat, Polyethylenglykol und einen wasserlöslichen Silikontyp enthält, getaucht und dann kurz mit Heißluft getrocknet. Anschließend wird zur Aufbringung des Handschuhes 3, die Tauchform 6 in eine vorvernetzte Naturlatexmischung getaucht.

Nach neuerlicher kurzer Trocknung wird er in ein Bad, welches einen geeigneten Acrylat-Copolymerlatex mit einem Trockengehalt von 10% enthält, getaucht, wobei die Gleitschicht 9 gebildet wird. Nach einem kurzen Trockenschritt wird zur Erzeugung der erfindungsgemäßen Rauheit kurz in ein Bad, welches 70% Methylethylketon und 30% Wasser enthält, getaucht. Danach wird mit Heißwasser gewaschen und 20 Minuten bei 120° C fertiggetrokknet. Zu Ende des Herstellungsprozesses wird der Handschuh 3 von der Form abgezogen, wobei die Außenseite nach innen gelangt und nachtrocknet.

Das Produkt hat eine Rauheit von etwa 10 µm und weist hervorragende Trageeigenschaften und sehr gute Anziehbarkeit sowohl mit trockenen als auch mit nassen Händen auf.

Dieses Verfahren ist auch für die Herstellung von puderfreien Syntheselatexhandschuhen geeignet, nur mit dem Unterschied, daß ein vorvernetzter Chloroprenlatex für die Herstellung der Trägerschicht 4 verwendet wird.

Selbstverständlich ist es möglich, daß die gesamte innere Oberfläche 12 des Handschuhs 3 nach den Fig. 2 und 3 mit den Gestaltsabweichungen 14 versehen ist, die die vorangegebene Rauhtiefe aufweisen.

Ebenso ist es möglich, daß die Unterseite 11 die äußere Oberfläche 12 der Trägerschicht 4 bildet, beispielsweise nur in dem die Handinnenfläche bildenden Teilbereich 7, 18 mit entsprechenden Gestaltsabweichungen 14, die gleich den zuvor angegebenen Werten der gemittelten Rauhtiefe hergestellt sein können, ausgebildet sein können. Der Vorteil der Anordnung dieser Rauheit nur auf der Handinnenseite liegt darin, daß damit ein Festkleben an Bauteilen oder Gegenständen bei Arbeitshandschuhen bzw. Werkzeugen in der Medizin verhindert wird, wogegen mit der Außenseite der Hand bzw. am Handrücken ein rutschsicheres Abstützen, wenn dies für die Kraftausbringung erforderlich ist, erzielt werden kann.

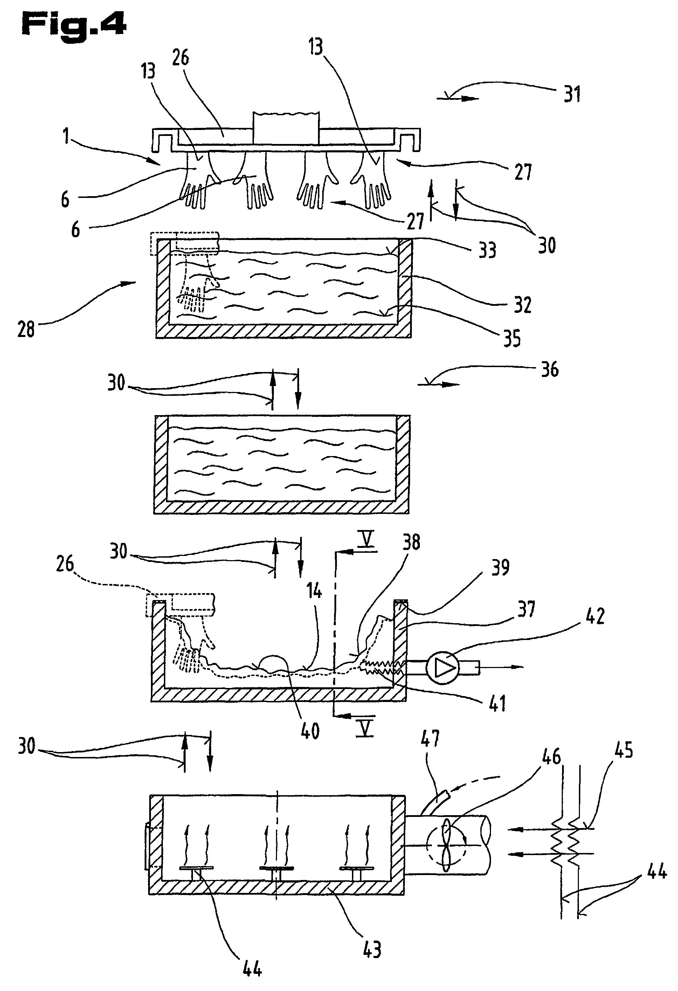

In den Fig. 4 bis 6 sind dann weitere Verfahrensmöglichkeiten zur Herstellung derartiger Gegenstände 1 und zum Herstellen der Gestaltsabweichungen 14 mit der vorangegebenen gemittelten Rauhtiefe gezeigt.

So ist es beispielsweise möglich, mit dem erfindungsgemäßen Verfahren puderfreie Latexoperationshandschuhe herzustellen. Dazu sind auf einem Formträger 26 mehrere Tauchformen 6, wie z.B. aus Porzellan, Kunststoff, Metall, Aluminium oder dgl., mit einer Gestaltsabweichung 14 aufweisenden Oberfläche 13 gemäß der vorangegebenen gemittelten Rauhtiefe versehen, während Tauchformen 27 mit einer glatten Oberfläche für die spätere Außenseite eines herzustellenden Handschuhs 3 aus Latex für Operationszwecke angeordnet sind.

Dem Formträger 26 ist ein Tauchbecken 28 zugeordnet, in dem sich eine wässrige Koagulationslösung 29 befindet. Diese kann, wie bereits einleitend angegeben, Kalziumnitrat, Polyethylenglykol und einen wasserlöslichen Silikontyp enthalten. Wie mit den Pfeilen 30 angedeutet, wird der Formträger 26 in das Tauchbecken 28 eingetaucht, dann aus diesem herausgenommen und gegebenenfalls nach einer Zwischentrocknung, wie symbolisch mit einem Pfeil 31 angedeutet, zu einem weiteren Tauchbecken 32 verbracht. Der Formträger 26 wird dann auch wieder in das Tauchbecken 32 abgesenkt, sodaß die Tauchformen 27 zur Gänze unter einen Flüssigkeitsspiegel 33 eintreten, wobei das Tauchbecken 32 mit einer Latexmischung sowohl auf Naturlatex als auch auf Syntheselatex basierend gefüllt sein kann, wobei sich diese sowohl unvernetzt als auch in vorvernetzten Zustand befinden kann.

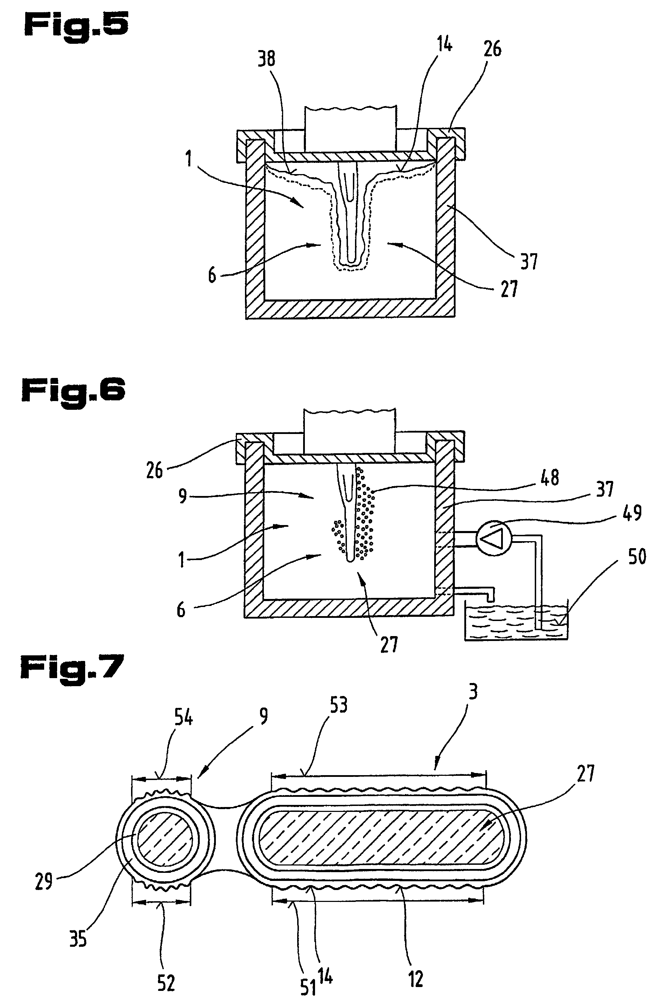

Anschließend an den durch die Pfeile 30 symbolisierten weiteren Tauchvorgang, der beispielsweise, um eine vorbestimmbare Wandstärke 34, wie in Fig. 3 ersichtlich, der Trägerschicht 4 festlegen zu können, auch mehrfach wiederholt werden kann, wird entweder eine im Tauchbecken 32 vorhandene Latexmischung 35 entleert, das Tauchbecken 32 gewaschen und das flüssige Rohmaterial zum Herstellen der Gleitschicht 9 eingebracht oder, wie dies mit Pfeile 36 angedeutet ist, gegebenenfalls nach einem kurzen Zwischentrocknungsvorgang und gegebenenfalls auch Waschvorgang der Formträger 26 in ein Formbecken 37 abgesenkt. Dieses Formbecken 37 ist im vorliegenden Fall mit einer hochelastischen Folie 38 versehen, welche dichtend am umlaufenden Rand 39 des Formbeckens 37 oder in sonst vergleichbarer technischer Weise befestigt ist. Das Aufsetzen des Formträgers 26 erfolgt derart, daß dieser einen dichten Innenraum zu einer Innenseite 40 der Folie 38 aufbaut. Diese Folie 38 ist, wie in Fig. 4 und zur besseren Darstellung in Fig. 5 in größerem Maßstab gezeigt, mit Gestaltsabweichungen 14 versehen und über eine bevorzugt flexible Schlauchleitung 41 mit dem Saugeingang einer Vakuumpumpe 42 verbunden. Ist nun der Formträger 26 auf das Formbecken 37 dichtend aufgesetzt, wird der von der Folie 38 und dem Formträger 26 umschlossene Hohlraum mit der Vakuumpumpe 42 evakuiert, da sich die Gestaltsabweichungen 14 der Folie 38 in die Gleitschicht 9 der auf den Tauchformen 6, 27 befindlichen Rohteile für die Handschuhe 3 bzw. Gegenstände 1 einprägen. Selbstverständlich ist es in diesem Zustand möglich, Wärme zuzuführen, um den Trockenvorgang bereits einzuleiten.

Anschließend an diese Behandlung wird entsprechend den Pfeilen 30 der Formträger 26 abgehoben und beispielsweise in eine Trockenkammer 43 verbracht, in der entweder durch in der Trockenkammer angeordnete Heizkörper 44 der Trockenvorgang gegebenenfalls nach einem vorhergehenden intensiven Waschvorgang fortgesetzt und abgeschlossen wird, wobei sich die Heizkörper 44, wie schematisch angedeutet, aber auch in einem Ansaugluftweg zum Lufttrocknen befinden können, wobei die zur Trocknung vorgesehene, durch Pfeile 45 schematisch angedeutet, Luft über einen Ventilator oder ein Gebläse 46 durch den Innenraum der Trockenkammer 43 hindurchgeführt werden kann. Selbstverständlich ist auch jede andere Form der Trocknung möglich. So auch beispielsweise die Trommeltrocknung oder dgl.. Bevorzugt ist es bei Verwendung einer derartigen Trockenkammer 43 möglich, über eine zusätzliche Leitung 47 eine Silikonemulsion in den Luftstrom gemäß dem dargestellten Pfeil einzublasen, um gegen Ende des Trocknungsvorganges oder am Schluß des Trocknungsvorganges, beispielsweise die die Innenseiten des Handschuhs 3 bildenden Oberflächen mit Silikon zu beschichten. Selbstverständlich ist es aber auch möglich, diese Silkonbeschichtung in ebenso bekannter Weise durch Tauchverfahren in Emulsionen oder durch Spritzauftrag in bestimmten Teilbereichen der Gleitzonen bzw. auf der Unterseite 11 bzw. der Oberfläche 12 aufzubringen.

Die erfindungsgemäße Rauheit kann aber auch durch viele andere bekannte Verfahren erzeugt werden.

Die einfachste Methode ist die Verwendung von aufgerauhten Formen bei der Herstellung von Gegenständen 1. Während dies beispielsweise bei der Herstellung im Spritzguß einfach zu bewerkstelligen ist, kann bei dünnwandigen Tauchartikeln nur die meist später an der Außenseite liegende Formseite der Gegenstände 1, also nur eine Seite, derart aufgerauht werden. Mechanisch aufgebrachte Rauhheiten werden meist durch Bestrahlung mit Sand, Glasperlen, Wasser u. a. erzeugt.

Bei Aufrauhung von Gegenständen 1 mit dieser Methode wird die mit dem noch weichen Gegenstand 1 bzw. der Gleitschicht 9 belegte Tauchform 6, 27 beispielsweise durch eine Sandstrahlkammer oder, wie in Fig. 6 gezeigt, durch ein Fließbett mit feinen Glasperlen 48 geführt. Überschüssige Partikel aus dem Strahlverfahren müssen üblicherweise in nachfolgenden Prozeßschritten vollständig entfernt werden.

Um die Wirkung dieser Glasperlen 48 oder sonstiger Aufrauhungskörper zu verstärken, ist es beispielsweise auch möglich, daß bei einem dichten Aufsetzen des Formträgers 26 auf das Formbecken 37 der Innenraum des Formbeckens 37, wie in Fig. 6 zusätzlich angedeutet, über eine Pumpe 49 mit einer unter Druck stehender Flüssigkeit 50 gefüllt wird, sodaß die Einprägewirkung in die noch nicht zur Gänze erhärtete Gleitschicht 9 stärker ist.

Selbstverständlich ist es aber auch möglich, die gemittelte Rauhtiefe durch eine chemische Aufrauhung herzustellen. Dazu wird die erfindungsgemäße Gleitschicht 9 in Latexform während des Tauchprozesses auf die Trägerschicht 4 bzw. den Gegenstand 1 aufgetragen und noch vor dem nachfolgenden Trocknungsschritt in ein geeignetes Lösungsmittel getaucht, in welchem das Gleitschichtpolymer anquillt. Nach anschließender Trocknung und Vulkanisation erhält man die erwünschte Rauheit. Werden wasserlösliche Lösungsmittel eingesetzt, kann durch das Mischverhältnis von Lösungsmittel zu Wasser der Rauhigkeitsgrad gut eingestellt werden. In diesem ist das Formbecken 37 in Fig. 4 ebenfalls als Tauchbecken 32 zur Aufnahme der Chemikalien bzw. des Lösungsmittels ausgebildet. Es erfolgt jedoch dann in diesem Becken der Formgebungsvorgang.

Des weiteren ist es aber auch möglich, in das Polymer 5 der Gleitschicht 9 fein gekörnte Füllstoffe einzubringen, sodaß diese vollständig vom Polymer 5 umschlossen werden. Hier kann durch die Teilchengröße und -menge die Rauheit gesteuert werden. Voraussetzung ist jedoch die gute Verträglichkeit des Füllstoffes mit dem Polymer 5, sodaß eine vollständige Benetzung und Überdeckung in der Gleitschicht 9 gegeben ist. Durch den vollständigen Einschluß der Teilchen in das Trägerpolymer wird ein Ablösen der eingeschlossenen Teilchen bei Dehnung des flexiblen Gegenstandes 1 verhindert.

Umgekehrt können auch Füllstoffe in der erfindungsgemäßen Gleitschicht 9 Verwendung finden, welche in einem nachfolgenden Arbeitsschritt wieder entfernt werden. Hier wird die Rauhheit über die entstehenden Fehlstellen erzeugt. Als Beispiel für diese Technik sei die Verwendung von Kreide als Füllstoff und deren Entfernung mit Säurewaschschritten erwähnt.

Von Vorteil ist es aber auch, nach dem erfindungsgemäßen Verfahren puderfreie PVC-Industriehandschuhe herzustellen.

Die zur Herstellung von PVC-Handschuhen verwendeten Tauchformen 6 aus Aluminium, welche erfindungsgemäß eine leichte Oberflächenrauheit mit einer gemittelten Rauhtiefe von 3 µm bis 5 µm aufweisen, werden vor dem Tauchprozeß gereinigt und getrocknet. Danach wird die Tauchform 6 in einem 90°C heißen Wasserbad temperiert und kurz mit Heißluft getrocknet. Anschließend wird zur Aufbringung eines Filmes die Tauchform 6 bei Raumtemperatur in eine PVC-Paste getaucht, welche neben Farbpigmenten und Stabilisatoren einen 50%igen Anteil an PVC-Granulat, suspendiert in einem Weichmacher (DOP), enthält. Sofort nach der Aufbringung des Handschuhfilmes auf die Form wird diese etwa 15 min bei 250°C in einen Heißluftofen gestellt. Hierbei tritt Diffusion des Weichmachers in die PVC-Partikel und Bildung eines homogenen Weich-PVC-Filmes auf (Gelierung). Direkt danach wird die noch heiße Form mit dem Handschuh kurz durch ein Fließbett mit Glasperlen 48 mit einem Durchmesser von etwa 100 µm geführt, wodurch die erfindungsgemäße Rauhung aufgebracht wird. Anschließend erfolgt Abspülen mit kaltem Wasser zur Abkühlung und Entfernung überschüssiger Glasperlen 48. Nach neuerlicher kurzer Trocknung des oberflächlichen Wassers wird das Produkt von der Tauchform 6 abgezogen (wobei die außen aufgetragene Rauhung an die Handschuhinnenseite gelangt). Der Handschuh 3 ist so vollkommen teilchenfrei und weist sehr gute Anziehbarkeit mit nassen und trockenen Händen auf. Auf die ansonsten notwendige und übliche Verwendung von Baumwollflocken oder Puder (Talkum) kann verzichtet werden.

Aber auch die Herstellung von Schwimmflossen ist gemäß dem erfindungsgemäßen Verfahren mit verbesserten Eigenschaften möglich.

Vielfach werden Schwimmflossen aus Naturkautschukmischungen im Formpreßverfahren hergestellt. Erfindungsgemäß ist hierbei die Spritzgußform so anzufertigen, daß diejenigen Formoberflächen, welche die Kontaktzone des Produktes mit dem Fuß (Schuhinnenteil) definieren, eine gemittelte Rauhtiefe von etwa 3 µm bis 7 µm aufweisen. Die Naturkautschukmischung wird in die Form eingebracht, heiß verpreßt und bei 130°C vulkanisiert. Nach der Entformung wird das Produkt entsprechend gewaschen und leicht oberflächenchloriert. Durch die auf das Produkt im Innenschuhbereich übertragene Aufrauhung ergibt sich eine wesentlich verbesserte Anziehbarkeit der Schwimmflossen, insbesondere mit nassen Füßen.

Schließlich ist aber auch die Herstellung von Operationshandschuhen aus Latex mit verbesserten Trageeigenschaften möglich. Dazu werden die dünnwandigen Latexhandschuhe durch Tauchen von Porzellanformen als Tauchformen 6, 27 in eine Naturlatexmischung in der erforderlichen Wandstärke hergestellt. Nach Aufbringung der Latexschicht werden die Handschuhe 3 bei 80°C mit Wasser ausgewaschen. Anschließend werden diese in ein Tauchbad mit folgender Zusammensetzung getaucht:
Acrylathaltiger Latex, Trockengehalt ca. 40 % = 30 Teile
Wärmesensibler Koagulant (Organopolysiloxan) = 0,1 Teile
Essigsäure 98 % = 0,2 Teile
Gemahlene Kieselerde, Teilchengröße 0,3 µm -10 µm = 2 Teile
Wasser = 67,7 Teile

Danach wird der Handschuh 3 ca. 25 Minuten in einem Heißluftofen bei 120°C getrocknet und vulkanisiert. Der fertige Handschuh 3 wird von der Tauchform 6, 27 abgezogen, nochmals gewaschen, zur Reduktion der Klebrigkeit an der Außenseite mit wenig Silikonemulsion behandelt und getrocknet.

Der Gegenstand weist hervorragende Trageeigenschaften, ausgezeichnete Schweißsaugfähigkeit und gute Anziehbarkeit sowohl mit nassen als auch mit trockenen Händen auf.

Schließlich ist in Fig. 7 anhand eines Schnittes durch einen auf einer Tauchform 27 angeordneten Handschuh 3 gezeigt, daß auf der beispielsweise glatt ausgebildeten Oberfläche im Tauchvorgang zuerst eine Koagulationslösung 29 und dann eine Latexmischung 35 aufgebracht wird, worauf die Gleitschicht 9 aufgetaucht bzw. aufgesprüht wird.

Durch eine entsprechende Ausbildung der Einrichtungen zum Einprägen der gemittelten Rauhtiefe bzw. der Gestaltsabweichung 14 in der Oberfläche 12 können beispielsweise dann, wenn dazu eine Folie 38, wie in der Fig. 4 und 5 ersichtlich ist, verwendet wird, die nicht extrem hochelastisch ist, diese Gestaltsabweichungen 14 nur in bestimmten Teilflächen 51, 52, 53, 54 angebracht sein, die sich beispielsweise nur über einen Großteil der horizontalen Anlageflächen des Gegenstandes 1 an der Hand des Benutzers erstrecken. Damit wird, ohne daß der gesamte Artikel zu stark geschwächt wird, eine ausreichende Naßschlüpfrigkeit, wie dies beispielsweise auch bei Schwimmflossen, Gummischuhen oder dgl. zweckmäßig sein kann, erreicht, da auch dort vielfach nur auf jenen Flächen, die eine Vorspannung zum festen Sitz des Gegenstandes 1 auf einen Körperteil des Benutzers einwirkt, eine hohe Gleitfähigkeit erzielt werden muß.

Diese in den vorhergehenden Figuren beschriebenen Varianten der Materialauswahl für die Trägerschicht 4 und die Gleitschichten 9 sind wahlweise einsetzbar. Selbstverständlich können die einzelnen vorstehend beschriebenen Ausführungsbeispiele und die in diesen Ausführungsbeispielen gezeigten Varianten und unterschiedlichen Ausführungen jeweils für sich eigenständige, erfinderische Lösungen bilden und beliebig miteinander kombiniert werden. Vor allem können die einzelnen in den Fig. 1, 2, 3, 4, 5; 6; 7 gekennzeichneten Ausführungen den Gegenstand von eigenständigen, erfindungsgemaßen Lösungen bilden. Die diesbezüglichen erfindungsgemäßen Aufgaben und Lösungen sind den Detailbeschreibungen dieser Figuren zu entnehmen.

Der Ordnung halber sei abschließend darauf hingewiesen, daß zum besseren Verständnis der Erfindung die einzelnen Ausführungsbeispiele teilweise unmaßstäblich verzerrt bzw. vergrößert dargestellt wurden. Es können auch einzelne Merkmale der in den einzelnen Ausführungsbeispielen gezeigten Merkmalskombinationen jeweils für sich eigenständige, erfindungsgemäße Lösungen bilden.

### Bezugszeichenaufstellung

- 1: Gegenstand
- 2: Latexmischung
- 3: Handschuh
- 4: Trägerschicht
- 5: Polymer

- 6: Tauchform
- 7: Teilbereich
- 8: Oberseite
- 9: Gleitschicht
- 10: Material

- 11: Unterseite
- 12: Oberfläche
- 13: Oberfläche
- 14: Gestaltsabweichung
- 15: Abstand

- 16: Abstand
- 17: Tiefe
- 18: Teilbereich
- 19: Einzelrauhtiefe
- 20: Einzelrauhtiefe

- 21: Einzelrauhtiefe
- 22: Einzelrauhtiefe
- 23: Einzelrauhtiefe
- 24: Einzelmeßstecke
- 25: Länge

- 26: Formträger
- 27: Tauchform
- 28: Tauchbecken
- 29: Koagulationslösung
- 30: Pfeil

- 31: Pfeil
- 32: Tauchbecken
- 33: Flüssigkeitsspiegel
- 34: Wandstärke
- 35: Latexmischung

- 36: Pfeil
- 37: Formbecken
- 38: Folie
- 39: Rand
- 40: Innenseite

- 41: Schlauchleitung
- 42: Vakuumpumpe
- 43: Trockenkammer
- 44: Heizkörper
- 45: Pfeil

- 46: Gebläse
- 47: Leitung
- 48: Glasperle
- 49: Pumpe
- 50: Flüssigkeit

- 51: Teilfläche
- 52: Teilfläche
- 53: Teilfläche
- 54: Teilfläche

## Patentansprüche

1. Gegenstand (1) mit einer Trägerschicht (4) aus Latex, der zumindest mit einem Teilbereich (7, 18) der Oberfläche (12) zur Berührung oder zur Anlage mit einer menschlichen Haut ausgebildet ist, insbesondere ein Handschuh (3), ein Kondom oder dgl., der in diesem Teilbereich (7, 18) der Oberfläche (12) mit einer Gleitschicht (9) aus einem polymeren Material (10) versehen ist, **dadurch gekennzeichnet, daß** zumindest eine Teilfläche (51 bis 54) des Teilbereiches (7, 18) eine diskrete, bevorzugt regelmäßig wiederkehrende, Gestaltsabweichung (14) der Oberfläche (12) aufweist, wobei das Verhältnis der Abstände (15, 16) der Gestaltsabweichung (14) zu ihrer Tiefe (17) zwischen 100 : 1 und 5 : 1 liegt und die gemittelte Rauhtiefe zwischen 0,5 µm und 100 µm, bevorzugt zwischen 1 µm und 40 µm beträgt.

2. Gegenstand (1) nach Anspruch 1, **dadurch gekennzeichnet, daß** der Latex ein vorvernetzter Natur- und/oder NBR-Latex ist.

3. Gegenstand (1) nach Anspruch 1 oder 2, **dadurch gekennzeichnet, daß** der Latex ein Polymer (5) enthält, welches leicht vernetzt ist.

4. Gegenstand (1) nach Anspruch 1 oder 2, **dadurch gekennzeichnet, daß** der Latex ein Polymer (5) enthält, welches vorvernetzt ist.

5. Gegenstand (1) nach Anspruch 3 oder 4, **dadurch gekennzeichnet, daß** das Polymer (5) stark hydrophile Gruppen, beispielsweise Acryl- bzw. Methacrylsäure, Acrylaten bzw. Methacrylaten, enthält.

6. Gegenstand (1) nach einem der Ansprüche 3 bis 5, **dadurch gekennzeichnet, daß** das Polymer (5) ein Gemisch von Monomerbausteinen und anderen stark hydrophilen bzw. polaren Polymerbausteinen, z.B. Vinylpyrollidon, Vinylalkohol, ist.

7. Gegenstand (1) nach Anspruch 6, **dadurch gekennzeichnet, daß** dem Gemisch der Monomerbausteine des Polymers (5) auch hydrophobe bzw. wenig Wasser aufnehmende Anteile, vorzugsweise Styrol, Butadien, Isopren und/oder Acrylnitril, zugesetzt sind.

8. Gegenstand (1) nach einem der Ansprüche 3 bis 7, **dadurch gekennzeichnet, daß** das Polymer (5) durch ein Polyurethan gebildet ist.

9. Gegenstand (1) nach Anspruch 1 oder 2, **dadurch gekennzeichnet, daß** der Latex ein Polymer (5) enthält, welches eine hohe Oberflächenadhäsion aufweist bzw. unvernetzt ist.

10. Gegenstand (1) nach einem der vorhergehenden Ansprüche, **dadurch gekennzeichnet, daß** die Trägerschicht (4) einen vorvernetzten Chloroprenlatex aufweist.

11. Gegenstand (1) nach einem der vorhergehenden Ansprüche, **dadurch gekennzeichnet, daß** zusätzlich zumindest ein weiterer Teilbereich auf einer äußeren, der Haut abgewandten Oberfläche des Gegenstandes (1), mit den Gestaltsabweichungen (14) versehen ist.

12. Gegenstand (1) nach einem der vorhergehenden Ansprüche, **dadurch gekennzeichnet, daß** die Gleitschicht (9) durch einen unvernetzten Naturkautschuk gebildet ist.

13. Gegenstand (1) nach einem der Ansprüche 1 bis 11, **dadurch gekennzeichnet, daß** die Gleitschicht (9) durch einen Syntheselatex bzw. ein Syntheselatexgemisch gebildet und an der Oberfläche (12) des Gegenstandes (1) fixiert ist.

14. Gegenstand (1) nach einem der vorhergehenden Ansprüche, **dadurch gekennzeichnet, daß** die Gleitschicht (9) ein Polyurethan enthält.

15. Gegenstand (1) nach einem der vorhergehenden Ansprüche, **dadurch gekennzeichnet, daß** die Gleitschicht (9) einen Acrylnitril-Butadienkautschuk enthält.

16. Gegenstand (1) nach einem der vorhergehenden Ansprüche, **dadurch gekennzeichnet, daß** die Gleitschicht (9) Polyacrylate oder Polymethacrylate enthält.

17. Gegenstand (1) nach einem der vorhergehenden Ansprüche, **dadurch gekennzeichnet, daß** die Gleitschicht (9) synthetische Polymere aufweist, welche wasseraufnehmende Gruppen aufweisen.

18. Gegenstand (1) nach einem der Ansprüche 1 bis 11 oder 13, **dadurch gekennzeichnet, daß** die Gleitschicht (9) durch einen Acrylat-Copolymerlatex mit einem Trockengehalt von 1% bis 20 %, bevorzugt 10 %, gebildet ist.

19. Gegenstand (1) nach einem der vorhergehenden Ansprüche, **dadurch gekennzeichnet, daß** zumindest auf die Bereiche der Gleitschicht (9), welche mit den Gestaltsabweichungen (14) versehen sind, ein Silikon aufgebracht ist.

20. Gegenstand (1) nach Anspruch 19, **dadurch gekennzeichnet, daß** die Menge des Silikons zumindest auf der Oberfläche der Teilbereiche (7, 18) zwischen 0,05 g/m² bis 3 g/m² , bevorzugt 0,25 g/m², beträgt.

21. Gegenstand (1) nach einem der vorhergehenden Ansprüche, **dadurch gekennzeichnet, daß** die gemittelte Rauhtiefe in Teilbereichen mit geringerer Relativbewegung zwischen dem Gegenstand (1) und der Haut eines Benutzers geringer ist.

22. Gegenstand (1) nach einem der vorhergehenden Ansprüche, **dadurch gekennzeichnet, daß** zumindest die Gleitschicht (9) an ihrer Oberfläche (12) chloriert ist.

23. Gegenstand (1) nach Anspruch 22, **dadurch gekennzeichnet, daß** die durch die Chlorierung aufgeworfenen, erhöhten Randbereiche der Oberflächenverformungen über die benachbarten Oberflächenzonen um eine Höhe von 0,5 µm bis 10 µm, bevorzugt < 1 µm, vorragen.

24. Gegenstand (1) nach einem oder mehreren der vorhergehenden Ansprüche, **dadurch gekennzeichnet, daß** dieser als medizinischer Latexhandschuh ausgebildet ist.

25. Verfahren zum Herstellen eines flexiblen Gegenstandes (1), insbesondere eines Handschuhes (3), eines Kondomes oder dgl., bei dem eine Trägerschicht (4) aus Latex hergestellt wird und diese in zumindest einem Teilbereich (7, 18) ihrer Oberfläche (12) mit einer Gleitschicht (9) aus einem polymeren Material (10) versehen wird und diese im Bereich ihrer von der Trägerschicht (4) abgewendeten Oberfläche mit einer Rauheit versehen wird, insbesondere zur Herstellung eines Gegenstandes (1) nach einem der vorhergehenden Ansprüche, **dadurch gekennzeichnet, daß** die Oberfläche (12) zumindest in Teilflächen (51 bis 54) des Teilbereiches (7, 18) mit diskreten, bevorzugt regelmäßig wiederkehrenden Gestaltsabweichungen (14) versehen wird, bei welchen das Verhältnis der Abstände (15, 16) der Gestaltsabweichungen (14) zu ihrer Tiefe (17) zwischen 100: 1 und 5: 1 liegt und deren gemittelte Rauhtiefe zwischen 1 µm bis 100 µm, bevorzugt 1 bis 40 µm, beträgt.

26. Verfahren nach Anspruch 25, **dadurch gekennzeichnet, daß** die Gestaltsabweichungen (14) durch Formflächen einer Tauchform (27) in der Trägerschicht (4) und/oder der Gleitschicht (9) ausgeformt werden.

27. Verfahren nach Anspruch 25, **dadurch gekennzeichnet, daß** die Gestaltsabweichungen (14) über eine unter Vakuum anlegbare elastische Folie (38) in die Oberfläche der Teilbereiche (7, 18) eingeprägt wird.

28. Verfahren nach Anspruch 25, **dadurch gekennzeichnet, daß** die Gestaltsabweichungen (14) durch eine Anpressung und einen Ausgleich der Dicke der aufgetragenen Schichten auf der Form mit aufgerauhter Oberfläche (13) durch Druckbelastung, beispielsweise mittels einer unter Druck stehenden Flüssigkeit, hergestellt werden.

29. Verfahren nach Anspruch 25, **dadurch gekennzeichnet, daß** die Gestaltsabweichungen (14) durch das Einpressen von Mikrokugeln oder durch Sandstrahlung hergestellt werden.

30. Verfahren nach einem der Ansprüche 25 bis 29, **dadurch gekennzeichnet, daß** auf die gerauhten Oberflächen der Teilbereiche (7, 18) ein Gleitmittel, insbesondere ein Silikon, aufgetragen wird.

31. Verfahren nach einem der Ansprüche 25 bis 30, **dadurch gekennzeichnet, daß** für die Gleitschicht (9) im Teilbereich (18) eine Mischung aus einem acrylhältigen Latex mit einem Trockengehalt von ca. 40 %, einem wärmesensiblen Koagulanten, z.B. ein Organopolysiloxan, Essigsäure, gemahlene Kieselerde, mit Teilchengrößen zwischen 0,3 µm bis 19 µm, und Wasser verwendet wird.

32. Verfahren nach Anspruch 25, **dadurch gekennzeichnet, daß** die Gestaltsabweichungen (14) durch das Eintauchen der aus Acrylatcopolymerlatex gebildeten Gleitschicht (9) in ein Bad aus 70 % Methylketon und 30 % Wasser hergestellt wird, worauf diese Gleitschicht (9) mit Heißwasser gewaschen und bei 120 °C fertig getrocknet wird.

33. Verfahren nach einem der Ansprüche 25 bis 32, **dadurch gekennzeichnet, daß** die Gestaltsabweichung (14) auf der äußeren Oberfläche (12) des Gegenstandes (1), insbesondere eines Gummi- bzw. Latexhandschuhs, durch Aufbringen des Materials für die Trägerschicht (4) auf eine Form, deren Oberfläche (13) Gestaltsabweichungen (14) aufweist, bei welchen das Verhältnis der Abstände (15, 16) der Gestaltsabweichung (14) zu ihrer Tiefe (17) zwischen 100 : 1 und 5 : 1 liegt und die gemittelte Rauhtiefe zwischen 0,5 µm und 100 µm, bevorzugt zwischen 1 µm und 40 µm, beträgt, hergestellt werden.

34. Verfahren nach Anspruch 26, **dadurch gekennzeichnet, daß** die Gestaltsabweichungen (14) an der inneren Oberfläche (12) des Handschuhs (3) bzw. in der Oberfläche der Gleitschicht (9) durch eine Druckverformung hergestellt werden, bei der eine gleichmäßige Dicke der Trägerschicht (4) und/oder Gleitschicht (9) hergestellt wird, sodaß die Rauheit der Formfläche der Tauchform (27) auf die äußere Oberfläche des Handschuhs (3) übertragen wird.

## Claims

1. Article (1) with a base layer (4) of latex, which is intended to be in contact with or lie against human skin at least with a part-region (7, 18) of the surface (12), in particular a glove (3), a condom or such like, this part-region (7, 18) of the surface (12) being provided with a slip coating (9) of a polymeric material (10), **characterised in that** at least a part-surface (51 to 54) of the part-region (7, 18) has a discrete, preferably regularly recurring structural variation (14) in the surface (12), wherein the ratio of the distances (15, 16) of the structural variation (14) to the depth (17) thereof is between 100 : 1 and 5 : 1 and the mean roughness depth is between 0.5 µm and 100 µm, preferably between 1 µm and 40 µm.

2. Article (1) as claimed in claim 1, **characterised in that** the latex is a cross-linked natural and/or NBR latex.

3. Article (1) as claimed in claim 1 or 2, **characterised in that** the latex contains a polymer (5) which is lightly cross-linked.

4. Article (1) as claimed in claim 1 or 2, **characterised in that** the latex contains a polymer (5) which is pre-cross-linked.

5. Article (1) as claimed in claim 3 or 4, **characterised in that** the polymer (5) contains highly hydrophobic groups, such as acrylic or methacrylic acid, acrylates or methacrylates, for example.

6. Article (1) as claimed in one of claims 3 to 5, **characterised in that** the polymer (5) is a mixture of monomer blocks and other highly hydrophilic or polar polymer blocks, e.g. vinyl pyrrolidone , vinyl alcohol.

7. Article (1) as claimed in claim 6, **characterised in that** quantities of substances which are hydrophobic or absorb little water, preferably styrene, butadiene, isoprene and/or acrylonitrile, are added to the mixture of monomer blocks of the polymer (5).

8. Article (1) as claimed in one of claims 3 to 7, **characterised in that** the polymer (5) is a polyurethane.

9. Article (1) as claimed in claim 1 or 2, **characterised in that** the latex contains a polymer (5), which has a high surface adhesion or is not cross-linked.

10. Article (1) as claimed in one of the preceding claims, **characterised in that** the base layer (4) contains a pre-cross-linked chloroprene latex.

11. Article (1) as claimed in one of the preceding claims, **characterised in that** at least another part-region on an external surface of the article (1) remote from the skin is additionally provided with the structural variations (14).

12. Article (1) as claimed in one of the preceding claims, **characterised in that** the slip coating (9) is a non-cross-linked natural rubber.

13. Article (1) as claimed in one of claims 1 to 11, **characterised in that** the slip coating (9) is a synthetic latex or a synthetic latex mixture and is fixed on the surface (12) of the article (1).

14. Article (1) as claimed in one of the preceding claims, **characterised in that** the slip coating (9) contains a polyurethane.

15. Article (1) as claimed in one of the preceding claims, **characterised in that** the slip coating (9) contains an acrylonitrile-butadiene rubber.

16. Article (1) as claimed in one of the preceding claims, **characterised in that** the slip coating (9) contains polyacrylate or polymethacrylate.

17. Article (1) as claimed in one of the preceding claims, **characterised in that** the slip coating (9) contains synthetic polymers containing water-absorbing groups.

18. Article (1) as claimed in one of claims 1 to 11 or 13, **characterised in that** the slip coating (9) is an acrylate-copolymer latex with a dry content of from 1% to 20%, preferably 10%.

19. Article (1) as claimed in one of the preceding claims, **characterised in that** a silicone is applied at least to the regions of the slip coating (9) provided with the structural variations (14).

20. Article (1) as claimed in claim 19, **characterised in that** the quantity of silicone applied at least to the surface of the part-regions (7, 18) is between 0.05 g/m² and 3 g/m², preferably 0.25 g/m².

21. Article (1) as claimed in one of the preceding claims, **characterised in that** the mean roughness depth is smaller in part-regions where there is less relative displacement between the article (1) and the skin of a user.

22. Article (1) as claimed in one of the preceding claims, **characterised in that** at least the slip coating (9) is chlorinated at its surface (12).

23. Article (1) as claimed in claim 22, **characterised in that** the heightened peripheral regions of the surface deformations raised by the chlorination process stand proud of the adjacent surface regions by a height of 0.5 µm to 10 µm, preferably < 1 µm.

24. Article (1) as claimed in one or more of the preceding claims, **characterised in that** it is a latex surgical glove.

25. Method of producing a flexible article (1), in particular a glove (3), a condom or such like, in which a base layer (4) of latex is produced and at least a part-region (7, 18) of its surface (12) is provided with a slip coating (9) of a polymeric material (10) and has a roughness imparted to the region of its surface remote from the base layer (4), in particular for producing an article (1) as claimed in one of the preceding claims, **characterised in that** the surface (12) is provided with discrete, preferably regularly recurring structural variations (14) at least in part-surfaces (51 to 54) of the part-region (7, 18), wherein the ratio of the distances (15, 16) of the structural variations (14) to the depth (17) thereof is between 100 : 1 and 5 : 1 and the mean roughness depth thereof is between 1 µm and 100 µm, preferably 1 to 40 µm.

26. Method as claimed in claim 25, **characterised in that** the structural variations (14) are formed in the base layer (4) and/or in the slip coating (9) by moulding surfaces of a dip mould (27).

27. Method as claimed in claim 25, **characterised in that** the structural variations (14) are imparted to the surface of the part regions (7, 18) by means of an elastic film (38) applied under vacuum.

28. Method as claimed in claim 25, **characterised in that** the structural variations (14) are produced by applying pressure in order to compress and differentiate the thickness of the applied coatings on the mould incorporating a roughened surface (13), for example by means of a fluid placed under pressure.

29. Method as claimed in claim 25, **characterised in that** the structural variations (14) are produced by pressing in micro-spheres or by sand-blasting.

30. Method as claimed in one of claims 25 to 29, **characterised in that** a lubricant, in particular a silicone, is applied to the roughened surfaces of the part-regions (7, 18).

31. Method as claimed in one of claims 25 to 30, **characterised in that** a mixture of an acrylic-containing latex with a dry content of approx. 40%, a heat-sensitive coagulant, e.g. an organo-polysiloxane, acetic acid, ground diatomaceous earth, with particle sizes of between 0.3 µm and 19 µm, and water are used for the slip coating (9) in the part-region (18).

32. Method as claimed in claim 25, **characterised in that** the structural variations (14) are produced by dipping the slip coating (9) of acrylate copolymer latex in a bath of 70% methyl ketone and 30% water, after which this slip coating (9) is washed with hot water and completely dried at 120°C.

33. Method as claimed in one of claims 25 to 32, **characterised in that** the structural variation (14) is imparted to the external surface (12) of the article (1), in particular a rubber or latex glove, by applying the material used for the base layer (4) to a mould, the surface (13) of which has structural variations (14), wherein the ratio of the distances (15, 16) of the structural variation (14) to its depth (17) is between 100 : 1 and 5 : 1 and the mean roughness depth is between 0.5 µm and 100 µm, preferably between 1 µm and 40 µm.

34. Method as claimed in claim 26, **characterised in that** the structural variations (14) on the internal surface (12) of the glove (3) or in the surface of the slip coating (9) are produced by compression moulding, which results in a uniform thickness of the base layer (4) and/or slip coating (9) so that the roughness of the moulding surface of the dip mould (27) is transferred to the external surface of the glove (3).

## Revendications

1. Objet (1) avec un support (4) en latex, qui est configuré au moins avec une zone partielle (7, 18) de la surface (12) pour un contact ou pour un appui avec une peau humaine, en particulier un gant (3), un condom ou analogue, qui est pourvu, dans cette zone partielle (7, 18) de la surface (12), d'une couche de glissement (9) en une matière polymère (10), **caractérisé en ce qu'**au moins une surface partielle (51 à 54) de la zone partielle (7, 18) présente un écart de configuration distinct (14), revenant préférentiellement régulièrement, de la surface (12), ce par quoi le rapport des distances (15, 16) de l'écart de configuration (14) à la profondeur (17) est compris entre 100 : 1 et 5 : 1 et la profondeur brute moyenne est entre 0,5 µm et 100 µm, préférentiellement entre 1 µm et 40 µm.

2. Objet (1) selon la revendication 1, **caractérisé en ce que** le latex est un latex naturel et/ou de NBR préréticulé.

3. Objet (1) selon la revendication 1 ou 2, **caractérisé en ce que** le latex contient un polymère (5), qui est facile à réticuler.

4. Objet (1) selon la revendication 1 ou 2, **caractérisé en ce que** le latex contient un polymère (5) qui est préréticulé.

5. Objet (1) selon la revendication 3 ou 4, **caractérisé en ce que** le polymère (5) contient des groupes forts hydrophiles, par exemple de l'acide acrylique ou respectivement méthacrylique, des acrylates ou respectivement méthacrylates.

6. Objet (1) selon l'une quelconque des revendications 3 à 5, **caractérisé en ce que** le polymère (5) est un mélange d'éléments monomères et autres éléments polymères fortement hydrophiles ou respectivement polaires, par exemple vinylpyrrolidone, alcool vinylique.

7. Objet (1) selon la revendication 6, **caractérisé en ce qu'**au mélange des éléments monomères du polymère (5) sont ajoutés également des constituants hydrophobes ou respectivement absorbant peu d'eau, avantageusement du styrène, du butadiène, de l'isoprène et/ou de l'acrylonitrile.

8. Objet (1) selon l'une quelconque des revendications 3 à 7, **caractérisé en ce que** le polymère (5) est formé d'un polyuréthanne.

9. Objet (1) selon la revendication 1 ou 2, **caractérisé en ce que** le latex contient un polymère (5) qui présente une forte adhérence de surface ou respectivement n'est pas réticulé.

10. Objet (1) selon l'une quelconque des revendications précédentes, **caractérisé en ce que** la couche de support (4) présente un latex de chloroprène préréticulé.

11. Objet (1) selon l'une quelconque des revendications précédentes, **caractérisé en ce qu'**au moins une autre zone partielle sur l'une des surfaces externes, éloignée de la peau, de l'objet (1), est pourvue des écarts de configuration (14).

12. Objet (1) selon l'une quelconque des revendications précédentes, **caractérisé en ce que** la couche de glissement (9) est formée d'un caoutchouc naturel non réticulé.

13. Objet (1) selon l'une quelconque des revendications 1 à 11, **caractérisé en ce que** la couche de glissement (9) est formée d'un latex de synthèse, ou respectivement d'un mélange de latex de synthèse et est fixée à la surface (12) de l'objet (1).

14. Objet (1) selon l'une quelconque des revendications précédentes, **caractérisé en ce que** la couche de glissement (9) contient un polyuréthanne.

15. Objet (1) selon l'une quelconque des revendications précédentes, **caractérisé en ce que** la couche de glissement (9) contient un caoutchouc d'acrylonitrile-butadiène.

16. Objet (1) selon l'une quelconque des revendications précédentes, **caractérisé en ce que** la couche de glissement (9) contient un polyacrylate ou un polyméthacrylate.

17. Objet (1) selon l'une quelconque des revendications précédentes, **caractérisé en ce que** la couche de glissement (9) présente des polymères synthétiques qui présentent des groupes absorbant l'eau.

18. Objet (1) selon l'une quelconque des revendications 1 à 11 ou 13, **caractérisé en ce que** la couche de glissement (9) est formée d'un latex d'un copolymère d'acrylate avec une teneur en matières sèches de 1 % à 20 %, préférentiellement de 10 %.

19. Objet (1) selon l'une quelconque des revendications précédentes, **caractérisé en ce qu'**au moins sur la zone de la couche de glissement (9) qui est pourvue des écarts de configuration (14) est appliquée une silicone.

20. Objet (1) selon la revendication 19, **caractérisé en ce que** la quantité de silicone, au moins sur la surface de la zone partielle (7, 18), est comprise entre 0,05 g/m² et 3 g/m², préférentiellement de 0,25 g/m².

21. Objet (1) selon l'une quelconque des revendications précédentes, **caractérisé en ce que** la profondeur brute moyenne dans les zones partielles avec un mouvement relatif minime entre l'objet (1) et la peau d'un utilisateur est minime.

22. Objet (1) selon l'une quelconque des revendications précédentes, **caractérisé en ce qu'**au moins la couche de glissement (9) est chlorée en surface (12).

23. Objet (1) selon la revendication 22, **caractérisé en ce que** les zones de bordure, surélevées par la chloruration, des déformations de surface, dépassent des zones de surface voisines d'une hauteur de 0,5 µm à 10 µm, préférentiellement < 1 µm.

24. Objet (1) selon l'une quelconque des revendications précédentes, **caractérisé en ce que** celui-ci est configuré comme un gant en latex médical.

25. Procédé pour la production d'un objet flexible (1), en particulier d'un gant (3), d'un condom ou analogue, où une couche de support (4) est fabriquée en latex et celle-ci est pourvue, au moins dans une zone partielle (7, 18) de sa surface (12), d'une couche de glissement (9) en un matériau polymère (10) et celle-ci est pourvue dans une zone de sa surface éloignée de la couche de support (4), d'une rugosité, en particulier pour la production d'un objet (1) selon l'une quelconque des revendications précédentes, **caractérisé en ce que** la surface (12), au moins dans les surfaces partielles (51 à 54) de la zone partielle (7, 18), est pourvue d'écarts de configuration (14) distincts, préférentiellement régulièrement récurrents, dans lesquels le rapport des distances (15, 16) des écarts de configuration (14) à la profondeur (17) est compris entre 100: 1 et 5 : 1 et dont la profondeur brute moyenne est comprise entre 1 µm et 100 µm, de préférence entre 1 et 40 µm.

26. Procédé selon la revendication 25, **caractérisé en ce que** les écarts de configuration (14) sont formés par les surfaces de formage d'un modèle mère (27) dans la couche de support (4) et/ou la couche de glissement (9).

27. Procédé selon la revendication 25, **caractérisé en ce que** les écarts de configuration (14) sont empreints sur une feuille élastique pouvant être appliquée sous vide (38) dans les surfaces des zones partielles (7, 18).

28. Procédé selon la revendication 25, **caractérisé en ce que** les écarts de configuration (14) sont produits par compression et compensation de l'épaisseur des couches appliquées sur la forme avec une surface (13) rendue rugueuse par charge sous pression, par exemple au moyen d'un liquide sous pression.

29. Procédé selon la revendication 25, **caractérisé en ce que** les écarts de configuration (14) sont produits en enfonçant des micro-billes ou par un jet de sable.

30. Procédé selon l'une quelconque des revendications 25 à 29, **caractérisé en ce que** sur les surfaces rugueuses des zones partielles (7, 18) est appliqué un agent de glissement, en particulier une silicone.

31. Procédé selon l'une quelconque des revendications 25 à 30, **caractérisé en ce que** pour la couche de glissement (9) dans la zone partielle (18), on utilise un mélange formé d'un latex acrylique avec une teneur en matières sèches d'environ 40 %, un agent coagulant sensible à la chaleur, par exemple un organopolysiloxane, de l'acide acétique, de la terre de silice broyée, avec une dimension de particules comprise entre 0,3 µm et 19 µm et de l'eau.

32. Procédé selon la revendication 25, **caractérisé en ce que** les écarts de configuration (14) sont produits par immersion de la couche de glissement (9) formée d'un latex d'un copolymère d'acrylate dans un bain constitué de 70 % de méthylcétone et de 30 % d'eau, et ensuite cette couche de glissement (9) est lavée à l'eau chaude et est totalement séchée à 120°C.

33. Procédé selon l'une quelconque des revendications 25 à 32, **caractérisé en ce que** l'écart de surface (14) sur la surface externe (12) de l'objet (1), en particulier d'un gant en caoutchouc ou en latex, est produit par application du matériau pour la couche de support (4) sur une forme, dont la surface (13) présente des écarts de configuration (14), où le rapport des distances (15, 16) de l'écart de configuration (14) à la profondeur (17) est compris entre 100 :1 et 5 : 1 et la profondeur moyenne brute est comprise entre 0,5 µm et 100 µm, préférentiellement entre 1 µm et 40 µm.

34. Procédé selon la revendication 26, **caractérisé en ce que** les écarts de configuration (14) sur la surface interne (12) du gant (3) ou respectivement dans la surface de la couche de glissement (9) sont provoqués par une déformation sous pression dans laquelle une épaisseur régulière de la couche de support (4) et/ou de la couche de glissement (9) est produite, de façon que la rugosité des surfaces de formage du modèle mère (27) soit transmise à la surface externe du gant (3).
